(19) Europäisches Patentamt
European Patent Office
Office européen des brevets

(11) Veröffentlichungsnummer: **0 301 270**
**A2**

(12) **EUROPÄISCHE PATENTANMELDUNG**

(21) Anmeldenummer: **88110626.4**

(22) Anmeldetag: **04.07.88**

(51) Int. Cl.⁴: **C07D 247/02 , C07D 233/32 , C07D 239/10 , C07D 405/06**

(30) Priorität: **25.07.87 DE 3724621**

(43) Veröffentlichungstag der Anmeldung: **01.02.89 Patentblatt 89/05**

(84) Benannte Vertragsstaaten: **DE FR GB IT**

(71) Anmelder: **BAYER AG**
**Konzernverwaltung RP Patentabteilung**
**D-5090 Leverkusen 1 Bayerwerk(DE)**

(72) Erfinder: **Köhler, Burkhard, Dr.**
**Virneburgstrasse 9**
**D-4150 Krefeld(DE)**
Erfinder: **Meyer, Rolf-Volker, Dr.**
**Buchheimer Strasse 23**
**D-4150 Krefeld(DE)**

(54) **Verfahren zur Herstellung von tetrasubstituierten cyclischen Harnstoffen und neue cyclische Harnstoffe.**

(57) Die Erfindung betrifft ein neues Verfahren zur Herstellung von tetrasubstituierten cyclischen Harnstoffen sowie neue cyclische Harnstoffe.

EP 0 301 270 A2

Xerox Copy Centre

# Verfahren zur Herstellung von tetrasubstituierten cyclischen Harnstoffen und neue cyclische Harnstoffe

Die Erfindung betrifft ein neues Verfahren zur Herstellung von tetrasubstituierten cyclischen Harnstoffen sowie neue cyclische Harnstoffe.

Tetrasubstituierte cyclische Harnstoffe können z.b. aus N,N'-Dialkyldiaminen und einem Carbonylreagenz wie Phosgen (z.B. Tetrahedron 21 (9), 2561-71 (1965)), einem Kohlensäurediethylester (z.B. Liebigs Ann. Chem. 232, 2241), Chlorcyan (z.B. US-P 2 689 249) oder Harnstoff (z.B. EP-OS 198 345) hergestellt werden.

Bei diesen Herstellungsmethoden muß für jeden neuen Harnstoff erst ein neues N,N'-Diakyldiamin hergestellt werden. Diese Diamine können z.B. aus 1,2-Dichlorethan und einem primären Amin hergestellt werden, wobei man mit großem Überschuß an primärem Amin arbeiten muß, um eine weitere Alkylierung des zunächst entstehenden N,N'-Dialkyldiamins mit 1,2-Dichlorethan zu den N,N'-Dialkylpiperazinen zurückzudrängen. Asymmetrisch substituierte N,N'-Dialkyldiamine lassen sich nur in statistischen Gemischen nach dieser Methode herstellen. Aus diesem Grund sind z.B. asymmetrisch substituierte cyclische Harnstoffe aus Diaminen und einer Carbonylkomponente kaum zugänglich.

Weiterhin sind die bei dieser Herstellungsmethode verwendeten Carbonylreagenzien entweder nur aufwendig handhabbar (z.B. Phosgen, Chlorcyan) oder so wenig reaktiv, daß bei hohen Temperaturen und/oder Drücken umgesetzt werden muß (z.B. Kohlensäurediethylester, Harnstoff).

Eine weitere Methode zur Herstellung von tetrasubstituierten cyclischen Harnstoffen ist die direkte Alkylierung der disubstituierten Harnstoffe mit Alkylhalogeniden unter Zusatz von Alkoholat (z.B. Khim. Geterotsikl Soedin 1973 (1), 90-93). Bei dieser Umsetzung werden mäßige Ausbeuten erreicht. Außerdem entstehen Salze als Nebenprodukte, was die Wirtschaftlichkeit des Verfahrens beeinträchtigt.

Eine breit anwendbare, mit guten Ausbeuten verlaufende Alkylierungsreaktion, die zu tetrasubstituierten cyclischen Harnstoffen führt, ist daher von großem Interesse.

Es wurde nun gefunden, daß sich unsubstituierte cyclische Harnstoffe mit Carbonylverbindungen wie aliphatischen oder aromatischen Aldehyden und Ketonen, in Gegenwart von Ameisensäure bei Temperaturen von 70 bis 220 °C, gegebenenfalls unter Zusatz einer starken schwer flüchtigen Säure, in guten Ausbeuten zu tetrasubstituierten cyclischen Harnstoffen umsetzen lassen.

Gegenstand der Erfindung ist daher ein Verfahren zur Herstellung von tetrasubstituierten Harnstoffen der Formeln (I) und (II)

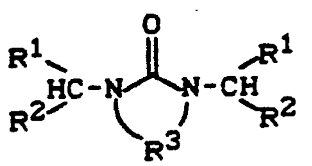 (I), 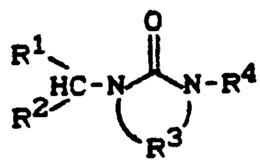 (II),

in welchen

$R^1$, $R^2$ unabhängig voneinander Wasserstoff, $C_1$-$C_{22}$ Alkyl bedeuten, mit der Maßgabe, daß die Summe der C-Atome aus beiden Resten $R^1$ und $R^2 \geq 4$ ist, für $C_5$-$C_{10}$-Cycloalkyl, $C_7$-$C_{20}$-Aralkyl, $C_7$-$C_{20}$-Alkaryl, $C_6$-$C_{14}$-Aryl und Furfuryl stehen, oder gemeinsam für $C_2$-$C_{12}$-Alkylen,

$R^3$ für $C_2$-$C_4$-Alkylen, das gegebenenfalls mit $C_1$-$C_6$-Alkyl substituiert ist oder für o-Phenylen, das gegebenenfalls mit einer Methylgruppe substituiert ist, steht und

$R^4$ für $C_1$-$C_{22}$-Alkyl, $C_5$-$C_7$-Cycloalkyl, $C_7$-$C_{20}$-Arylalkyl, $C_7$-$C_{20}$-Alkylaryl und $C_6$-$C_{14}$-Aryl steht,

dadurch gekennzeichnet, daß Carbonylverbindungen der Formel (III)

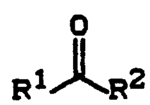 (III),

in welcher

$R^1$ und $R^2$ die bei den Formeln (I) und (II) angegebene Bedeutung haben,

mit Harnstoffen der Formel (VI) und (V)

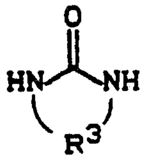 (IV), 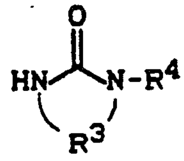 (V),

in welcher
$R^3$ und $R^4$ die bei Formeln (I) und (II) angegebene Bedeutung haben,
in Gegenwart von Ameisensäure bei einer Temperatur von 70 bis 220 °C umgesetzt werden.

Die Umsetzung dauert 1 bis 100, vorzugsweise 10 bis 40 Stunden. Die Temperatur beträgt, vorzugsweise 100 bis 140 °C.

Die Reste $R^1$ und $R^2$ bedeuten vorzugsweise Wasserstoff, $C_3$-$C_8$-Alkyl, Phenyl und Furfuryl, besonders bevorzugt Wasserstoff und $C_4$-Alkyl.

$R^3$ steht vorzugsweise für $C_2$-$C_4$-Alkylen, das gegebenenfalls mit einer Methylgruppe substituiert ist.

Bevorzugt werden Aldehyde und Ketone der Formel (VI)

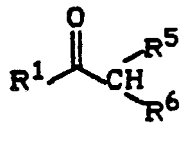 (VI),

in welcher
$R^1$ für Wasserstoff oder $C_1$-$C_4$-Alkyl, vorzugsweise H oder $CH_3$ steht und
$R^5$ und $R^6$ unabhängig voneinander $C_1$-$C_5$-Alkyl, vorzugsweise Methyl bedeuten,
eingesetzt.

Der Rest $R^4$ in Formeln (III) und (V) steht für $C_1$-$C_{22}$-Alkyl, bevorzugt Methyl, $C_5$-$C_7$-Cycloalkyl, $C_7$-$C_{20}$-Aralkyl oder $C_6$-$C_{14}$-Aryl, bevorzugt Phenyl.

Beispiele für Carbonylverbindungen der Formel (III) sind gesättigte aliphatische Aldehyde mit 4 bis 22 C-Atomen wie Butyraldehyd, Isobutyraldehyd, Valeraldehyd, 2-Ethylhexanal-1, Trichloracetaldehyd und Trichlorpropionaldehyd; cycloaliphatische Aldehyde vorzugsweise mit 5 bis 7 Ringgliedern wie Formylcyclohexan; araliphatische Aldehyde wie Phenylacetaldehyd und Zimtaldehyd; aromatische Aldehyde wie Benzaldehyd, o-, m- und p-Chlorbenzaldehyd, o-, m- und p-Methylbenzaldehyd, p-Methoxybenzaldehyd, α-Naphthaldehyd und ß-Napthaldehyd; aliphatisch-aliphatische Ketone wie Ethylbutylketon, Methylisobutylketon und Methylisopropylketon; aliphatischcycloaliphatische Ketone vorzugsweise mit 5 bis 12 Ringgliedern im Cycloalkylrest wie Methylcyclohexylketon; aliphatisch-araliphatische Ketone wie Phenylaceton; aliphatisch-aromatische Ketone wie Acetophenon, Propiophenon, p-Methylacetophenon, p-Chloracetophenon und Phenylethylketon; cycloaliphatisch-cycloaliphatische Ketone vorzugsweise mit 5 bis 6 Ringgliedern in den Cycloalkylresten wie Dicyclohexylketon; cycloaliphatisch-araliphatische Ketone wie Cyclohexylbenzylketon; cycloaliphatisch-aromatische Ketone wie Phenylcyclohexylketon; araliphatisch-araliphatische Ketone wie Dibenzylketon; araliphatisch-aromatische Ketone wie Phenylbenzylketon; aromatisch-aromatische Ketone wie Benzophenon sowie iso- und heterocyclische Ketone mit bevorzugt 5 bis 12 Ringgliedern wie Cyclopentanon, Cyclohexanon, Methylcyclohexanon, Cyclooctanon und Cyclododecanon.

Beispiele für Harnstoffe der Formel (IV) sind 5-Ring-Harnstoffe, z.B. Imidazolidinon-2, 4-Methylimidazolidinon-2, 3,4-Benzoimidazolidinon-2, 3,4-(4′-Methylbenzo)imidazolidinon-2; 6-Ring-Harnstoffe, z.B. Tetrahydropyrimidinon-2.

Beispiele für die Harnstoffe der Formel (V) sind 5-Ring-Harnstoffe, z.B. N-Methylimidazolidinon-2, 1,4-Dimethylimidazolidinon-2; 6-Ring-Harnstoffe, z.B. N-Methyl-tetrahydropyrimidinon-2.

Man führt die Umsetzung bei Temperaturen von 70 bis 220 °C aus, vorzugsweise bei 100 bis 140 °C, vorzugsweise bei Normaldruck.

Die Harnstoffe reagieren mit den Carbonylverbindungen in stöchiometrischen Mengen. Es kann jedoch vorteilhaft sein, die Carbonylkomponente in bis zu zehnfachem molaren Überschuß einzusetzen.

Die Ameisensäure wird in molaren bis zu 20-fach molaren, vorzugsweise 3 bis 10-fach molaren Mengen, bezogen auf Menge an Harnstoff eingesetzt. Die Reaktionszeit beträgt 1 bis 100 Stunden, bevorzugt 10 bis 40 Stunden.

Man kann die Reaktion mit und ohne Lösungsmittel, vorzugsweise ohne Lösungsmittel, ausführen. Als Solventien eignen sich inerte organische Flüssigkeiten, die einen Siedepunkt ≥75 °C haben, z.B. Cyclohexan, Benzol, Dimethoxyethan, Dioxan, Toluol, Xylol, Mesitylen, Diethylenglykoldimethylether, Tetralin u.s.w..

Die Aufarbeitung der Umsetzungslösung erfolgt durch Abdestillieren überschüssiger Reaktionspartner und des gegebenenfalls eingesetzten Lösungsmittels. Der Rückstand wird auf übliche Weise destilliert, gegebenenfalls im Vakuum, oder, falls es sich um einen Feststoff handelt, auf übliche Weise umkristallisiert.

Gegenstand der Erfindung sind auch neue tetrasubstituierte cyclische Harnstoffe der Formel (VII)

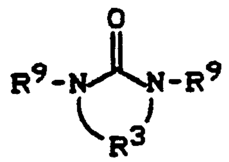

(VII),

in welcher $R^3$ die bei Formeln (I) und (II) angegebene Bedeutung hat,
die Reste $R^9$ unabhängig voneinander für $R^4$ und die Formel (VIIa)

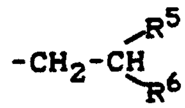

(VIIa), stehen,

in welcher die Reste
$R^4$, $R^5$ und $R^6$ unabhängig voneinander sind und die bei Formeln (II) und (VI) angegebene Bedeutung haben.

Bevorzugte Reste der Formel (VIIa) sind Isobutyl und 2-Ethyl-hexyl.

Bevorzugte Harnstoffe entsprechen der Formel (VIII)

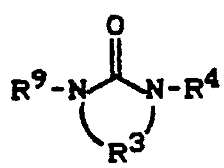

(VIII),

wobei
$R^9 \neq R^4$ sein muß und $R^9$ die bei Formel (VII), $R^3$ und $R^4$ die bei Formel (II) angegebene Bedeutung haben.

Bei dem erfindungsgemäßen Verfahren handelt es sich um eine Methode zur Alkylierung cyclischer Harnstoffe, die unter milden Reaktionsbedingungen verläuft, hohe Ausbeuten liefert und vielseitig anwendbar ist. Es lassen sich von einem einzigen cyclischen Harnstoff ausgehend durch Wahl verschiedener Aldehyde oder Ketone verschiedene tetrasubstituierte, cyclische Harnstoffe herstellen. Auch unsymmetrisch am Stickstoff substituierte, cyclische Harnstoffe sind nunmehr leicht zugänglich.

Die erfindungsgemäß zugänglichen tetrasubstituierten, cyclischen Harnstoffe eignen sich u.a. als Solventien für nucleophile Austauschreaktionen sowie bevorzugt für Polykondensationen, die vorteilhaft bei Temperaturen >230° C durchgeführt werden.

## Beispiele

### Beispiel 1

Man erhitzt die Lösung von 860 g (10 Mol) Imidazolidinon-2 mit 1775 g (25 Mol) Isobutyraldehyd in 3 l Ameisensäure 30 h bei 110° C unter Rückfluß. Anschließend destilliert man die überschüssigen Reaktionspartner bei Normaldruck ab. Nach Destillation erhält man 1650 g N,N′-Diisobutylimidazolidinon-2 als eine schwach gelb gefärbte Flüssigkeit vom Siedepunkt 110° C/0,5 Torr (Schmelzpunkt 6 bis 8° C).

Beispiel 2

Man erhitzt die Lösung von 10 g (0,1 Mol) 4-Methylimidazolidinon-2 mit 17,75 g (0,25 Mol) Isobutyraldehyd in 30 ml Ameisensäure 30 h bei 110 °C unter Rückfluß. Anschließend destilliert man die überschüssigen Reaktionspartner bei Normaldruck ab. Nach Destillation erhält man 10,5 g 1,3-Diisobutyl-4-methyl-imidazolidinon-2 vom Siedepunkt 117 °C/0,5 Torr.

Beispiel 3

Man erhitzt die Lösung von 10 g (0,1 Mol) Tetrahydropyrimidinon-2 mit 17,75 g (0,25 Mol) Isobutyraldehyd in 30 ml Ameisensäure 30 h bei 110 °C unter Rückfluß. Anschließend destilliert man die überschüssigen Reaktionspartner bei Normaldruck ab. Nach Destillation erhält man 12,3 g 1,3-Diisobutyltetrahydropyrimidinon-2 vom Siedepunkt 123 °C/0,5 Torr.

Beispiel 4

Man erhitzt die Lösung von 8,6 g (1 Mol) Imidazolidinon-2 mit 245 g (2,5 Mol) Cyclohexanon in 300 ml Ameisensäure 30 h bei 120 °C unter Rückfluß. Anschließend destilliert man die überschüssigen Reaktionspartner erst bei Normaldruck, dann im Wassrstrahlpumpenvakuum ab. Nach Destillation erhält man 117 g N,N′-Dicyclohexylimidazolidinon-2 vom Siedepunkt 170 °C/0,5 Torr.

Beispiel 5

Man versetzt 880 g (10 Mol) 1-Amino-3-methylaminopropan mit 600 g (10 Mol) Harnstoff und erhitzt 4 Stunden auf 150 °C und weitere 4 Stunden auf 200 °C. Man läßt auf 110 °C abkühlen, gibt 2,3 l (50 Mol) Ameisensäure und 1462 g (17 Mol) Isobutyraldehyd zu und erhitzt 30 Stunden auf 110 °C. Man destilliert die überschüssigen Reaktionspartner ab und erhält nach Destillation im Vakuum 1356 g 1-Isobutyr-3-methyltetrahydropyrimidinon-2 vom Siedepunkt 170 °C/25 Torr.

Beispiel 6

Man versetzt 43 g (0,5 Mol) Imidazolidinon-2 mit 172 g (1,1 Mol) 2-Ethylhexanal und 300 ml (6,5 Mol) Ameisensäure und erhitzt 30 Stunden auf 110 °C. Man destilliert die überschüssigen Reaktionspartner ab und erhält nach Destillation im Vakuum 155 g N,N′-Di-2′-ethylhexyl-1′-imidazolidinon-2 vom Siedepunkt 150 °C/1 Torr.

**Ansprüche**

1. Verfahren zur Herstellung von tetrasubstituierten Harnstoffen der Formeln (I) und (II)

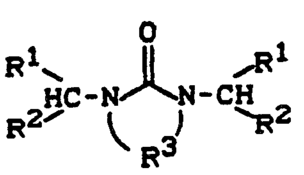 (I),

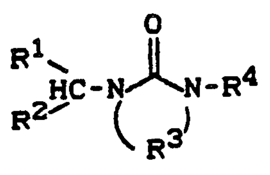 (II),

in welchen

$R^1$, $R^2$ unabhängig voneinander Wasserstoff, $C_1$-$C_{22}$ Alkyl bedeuten, mit der Maßgabe, daß die Summe der C-Atome aus beiden Resten $R^1$ und $R^2$ ≥ 4 ist, für $C_5$-$C_{10}$-Cycloalkyl, $C_7$-$C_{20}$-Aralkyl, $C_7$-$C_{20}$-Alkaryl, $C_6$-$C_{14}$-Aryl und Furfuryl stehen, oder gemeinsam für $C_2$-$C_{12}$-Alkylen,

$R^3$ für $C_2$-$C_4$-Alkylen, das gegebenenfalls mit $C_1$-$C_6$-Alkyl substituiert ist oder für o-Phenylen, das gegebenenfalls mit einer Methylgruppe substituiert ist, steht und

$R^4$ für $C_1$-$C_{22}$-Alkyl, $C_5$-$C_7$-Cycloalkyl, $C_7$-$C_{20}$-Arylalkyl, $C_7$-$C_{20}$-Alkylaryl und $C_6$-$C_{14}$-Aryl steht, dadurch gekennzeichnet, daß Carbonylverbindungen der Formel (III)

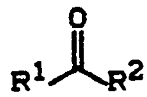 (III),

in welcher
$R^1$ und $R^2$ die bei den Formeln (I) und (II) angegebene Bedeutung haben,
mit Harnstoffen der Formel (VI) und (V)

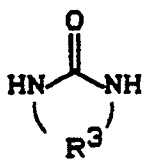 (IV), 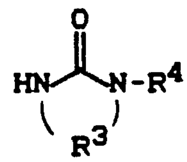 (V),

in welcher
$R^3$ und $R^4$ die bei Formeln (I) und (II) angegebene Bedeutung haben,
in Gegenwart von Ameisensäure bei einer Temperatur von 70 bis 220 °C umgesetzt werden.

2. Harnstoffe der Formel (VII)

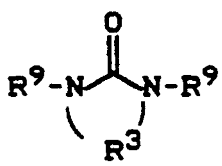 (VII),

in welcher $R^3$ die in Anspruch 1 angegebene Bedeutung hat,
die Reste $R^9$ unabhängig voneinander für $R^4$ und die Formel (VIIa)

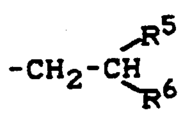 (VIIa), stehen,

in welcher die Reste
$R^4$, $R^5$ und $R^6$ unabhängig voneinander sind und die in Anspruch 1 angegebene Bedeutung haben.

3. Harnstoffe der Formel (VIII)

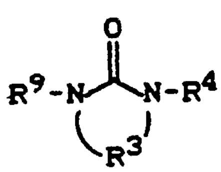 (VIII),

wobei
$R^9 \neq R^4$ sein muß und $R^9$ und $R^4$ die in Anspruch 1 angegebene Bedeutung haben.